# EUROPEAN PATENT APPLICATION

(11) **EP 2 380 561 A1**
(43) Date of publication of application: **26.10.2011**
(21) Application number: 10160642.4
(22) Date of filing: 21.04.2010
(51) Int. Cl.: A61K 9/20, A61K 31/404

(54) **Extended release formulation comprising indapamide**

(71) Applicant: LEK Pharmaceuticals d.d., 1526 Ljubljana (SI)
(72) Inventor: Klancar, Uros, 1526 Ljubljana (SI); Polona, Jurkovic, 1526 Ljubljana (SI); Igor, Legen, 1526 Ljubljana (SI); Matej, Horvat, 1526 Ljubljana (SI)
(74) Representative: Kunic Tesovic, Barbara

(57) **Abstract**

The present invention relates to an extended release pharmaceutical composition comprising indapamide as an active ingredient and a cellulose derivative, the process of manufacturing the composition and its use as a medicament in mammals.

## Description

### FIELD OF THE INVENTION

The present invention relates to an extended release pharmaceutical composition comprising indapamide, the process of manufacturing the composition and its use as a medicament in mammals.

### BACKGROUND OF THE INVENTION

Diuretics are commonly used as drugs in the therapy of congestive heart failure, hypertension, liver cirrhosis and kidney diseases. One major objective for diuretic drug design is a formulation ensuring constant plasma levels while at the same time allowing for once-daily administration. An ideal extended release (ER) formulation is hence characterized by a release profile following zero order kinetics, thus avoiding immediate drug release and maintaining plasma concentrations in the therapeutic range for a longer period. Patient compliance generally increases with reduced number of daily administrations, which helps to reduce fluctuations in the plasma levels of a drug that has to be administered less frequently. Indapamide, which is widely used for treatment of congestive heart failure and hypertension, has initially been employed as an immediate release (lR) pharmaceutical at a daily dose of 2.5 mg. Further development led to extended release formulations of indapamide, which are administered at a single daily dose of 1.5 mg. The reduction of total drug amount and the more beneficial pharmacokinetics of the ER formulation have been demonstrated to increase tolerability of the treatment without compromising the therapy's efficacy as compared to a treatment using 2.5 mg indapamide lR per day.

Several formulations have been described in prior art with the objective of providing for an extended release profile of indapamide. US 5,334,392 describes indapamide ER formulations comprising a hydrophilic matrix of two polymers wherein one is a cellulose derivative and the other is povidone. The cellulose derivative practically contemplated in US 5,334,392 is hydropropylmethylcellulose (HPMC) having a viscosity around 4000 cps (see US 5,334,392, table 3). Similar compositions manufactured via both, wet-granulation and direct compression processes, are described in Damien et al. (Clin. Pharmacokinet. 1999; 37 Suppl. 1: 13-19) and WO2005/074884A2. EP1902709A1 and EP1669062A1 teach that it is not necessary to combine two polymers from two different chemical families in order to achieve extended release of indapamide from tablets. Compositions are described therein where cellulose polymers of different viscosities are combined. Two HPMC polymers of different viscosities are also employed in the compositions described in Sheng-Miao et al. (Chin. Hosp. Pharm. J. 2003 Jul. Vol 23, No. 7).

However, to date no indapamide ER formulation is known, where the release of indapamide from the dosage form follows zero order kinetics under the mechanical stress conditions encountered in the gastrointestinal tract and it cannot be regarded as sufficient to measure the drug release from a dosage form *in vitro.*

Hence, although indapamide formulations already exist, there is still a need for a composition that ensures improved extended release properties. It is therefore an object of the present invention to provide a pharmaceutical composition comprising indapamide, which enables extended drug release under mechanical stress in the gastrointestinal environment.

### SUMMARY OF THE INVENTION

The present invention provides the following aspects, subject-matters and preferred embodiments, which respectively taken alone or in combination, further contribute to solving the object of the present invention:
(1) An extended release pharmaceutical composition comprising indapamide as an active ingredient and a cellulose derivative, wherein the viscosity of the cellulose derivative contained in the pharmaceutical composition is equal to or above 11250 cps as determined in a 2% aqueous solution at 20 °C following the standard protocol according to the US Pharmacopeia USP29.
(2) An extended release pharmaceutical composition comprising indapamide as an active ingredient and as pharmaceutical excipients at least microcrystalline cellulose and a cellulose derivative, wherein the viscosity of the cellulose derivative contained in the pharmaceutical composition is equal to or above 11250 cps as determined in a 2% aqueous solution at 20 °C following the standard protocol according to the US Pharmacopeia USP29.
(3) An extended release pharmaceutical composition according to item 1, further comprising microcrystalline cellulose.
(4) An extended release pharmaceutical composition according to any of the preceding items, wherein the cellulose derivative contained in the pharmaceutical composition has a viscosity from 11250 cps to 19500 cps as determined in a 2% aqueous solution at 20 °C following the standard protocol according to the US Pharmacopeia USP29.
(5) The pharmaceutical composition according to any of the preceding items, wherein the cellulose derivative is selected to have a nominal viscosity of about 15000 cps or about 100000.
(6) The pharmaceutical composition according to any of the preceding items, wherein the cellulose derivative is a high-viscosity grade hydroxypropylmethylcellulose having a nominal viscosity of 15000 cps according to the US Pharmacopeia USP29 as commercially available as HPMC Type 2208, grade K15M; or the cellulose derivative is a high-viscosity grade hydroxypropylmethylcellulose having a nominal viscosity of 100000 cps according to the US Pharmacopeia USP29 as commercially available as HPMC Type 2208, grade K100M.
(7) The pharmaceutical composition according to any of the preceding items, wherein the cellulose derivative is a high-viscosity grade hydroxypropylcellulose having a viscosity of 1% aqueous solution between 1500 and 3000 mPas using Brookfield method, which corresponds to a viscosity above 11250 cps if determined in a 2% aqueous solution at 20 °C following the standard protocol according to the US Pharmacopeia USP29, as commercially available as Klucel Type HF or Type HXF.
(8) The pharmaceutical composition according to any of the preceding items, which is characterized by extended drug release under gastrointestinal stress, wherein at least 70% of the indapamide present in the dosage form are not released before 2 hours.
(9) The pharmaceutical composition according to any of the preceding items, which is characterized by extended drug release following mechanical stress, wherein at least 70% of the indapamide present in the dosage form are not released before 2 hours, as determined by an extended basket method including a manipulation step for simulating gastrointestinal mechanical stress.
(10) The pharmaceutical composition according to any of the preceding items, which is characterized by extended drug release following mechanical stress such that, when mechanical stress was applied for a limited period of time, the maximum dissolution rate remains less than 175%, preferably less than 150% when compared to the dissolution rate before the application of mechanical stress.
(11) The pharmaceutical composition according to any of the preceding items, wherein the relative amount of the cellulose derivative having a viscosity equal to or above 11250 cps as determined in a 2% aqueous solution at 20 °C following the standard protocol according to the US Pharmacopeia USP29, is between 20 and 70% weight/weight of the tablet core, more preferably between 30 and 60%, even more preferably between 35 and 50%.
(12) The pharmaceutical composition according to any of the preceding items, comprising further pharmaceutical excipients selected from fillers, binders and lubricants.
(13) The pharmaceutical composition according to any of the preceding items, further comprising povidone.
(14) The pharmaceutical composition according to any of the preceding items, wherein a further excipient is povidone and the weight ratio between the cellulose derivative having a viscosity equal to or above 11250 cps as determined in a 2% aqueous solution at 20 °C following the standard protocol according to the US Pharmacopeia USP29 and povidone is between 1:0.30 and 1:0.03, preferably between 1:0.17 and 1:0.04.
(15) The pharmaceutical composition according to any of the preceding items, wherein a further excipient is microcrystalline cellulose and the weight ratio between the cellulose derivative having a viscosity equal to or above 11250 cps as determined in a 2% aqueous solution at 20 °C following the standard protocol according to the US Pharmacopeia USP29 and the microcrystalline cellulose is preferably between 1:2 and 1:0.07, preferably between 1:1.2 and 1:0.1.
(16) The pharmaceutical composition according to any of the preceding items, further comprising lactose monohydrate, colloidal anhydrous silica and magnesium stearate.
(17) The pharmaceutical composition according to any of the preceding items, further comprising excipients providing for pleasant taste and/or colour.
(18) The pharmaceutical composition according to any of the preceding items comprising one or more coating layers.
(19) The pharmaceutical composition according to any of the preceding items comprising a second active ingredient.
(20) A process for manufacturing the pharmaceutical composition according to any of the preceding items comprising mixing indapamide and a cellulose derivative having a viscosity equal to or above 11250 cps as determined in a 2% aqueous solution at 20 °C following the standard protocol according to the US Pharmacopeia USP29.
(21) The process according to item 20, wherein the cellulose derivative is blended with granules comprising indapamide as the active ingredient before compression into tablets.
(22) The pharmaceutical composition according to any of items 1 to 19 for use as a medicament in mammals.
(23) The pharmaceutical composition according to item 22 for use as a medicament in treatment or prophylaxis of diseases selected from the group consisting of heart failure including also congestive heart failure; hypertension; liver cirrhosis; hypercalciuria; cardiovascular event risk in diabetes mellitus including also diabetes mellitus type 2; edema; ischemic reperfusion injury; neurohypophyseal diabetes insipidus; Raynaud's phenomenon and systolic hypertension-Isolated.
(24) The pharmaceutical composition according to item 22, wherein the pharmaceutical composition is used alone or in combination with another medicament.
(25) The pharmaceutical composition according to item 24, wherein the other medicament is an antihypertensive drug from groups of angiotensin converting enzyme (ACE) inhibitors, angiotensin II receptor blockers, beta blockers or calcium channel blockers.

### BRIEF DESCRIPTION OF THE DRAWINGS

- Figure 1:: cumulative amount of drug released (in percent) from the compositions described in Examples 1, 4, 5 and Comparative Examples 2 and 3 plotted versus time (in hours)
- Figure 2:: cumulative amount of drug released (in percent) from the compositions described in Examples 1, 4, 5 and Comparative Examples 2 and 3 plotted versus time (in hours): magnification of burst release area
- Figure 3:: rates of dissolution of Examples 1, 4, 5 and Comparative Examples 2 and 3

### DESCRIPTION OF THE PREFERRED EMBODIMENTS/GENERAL DESCRIPTION

The present invention is now described in more detail by preferred embodiments and examples, which are however presented for illustrative purpose only and shall not be understood as limiting the scope of the present invention in any way.

According to the present invention a pharmaceutical composition comprising indapamide or pharmaceutically acceptable salts thereof is provided. Employing modified drug release assays it has surprisingly been found that the use of particular cellulose derivatives having a certain minimum viscosity, notably above 11250 cps (determined according to USP29 as 2% solution at 20 °C) enables robust extended release from the pharmaceutical composition under mechanical and chemical stress conditions as encountered in the gastrointestinal tract. Under physiological conditions an orally administrated dosage form undergoes considerable mechanical stress during its passage through the gastrointestinal tract, in particular during transition through the pylorus. An insufficiently formulated dosage form will result in rapid disintegration of the tablet leading to sudden drug release usually referred to as 'burst release'. In order to simulate the conditions leading to this burst, an additional step has been introduced into the standard protocol of the so-called basket method, which is widely used for measuring drug release from dosage forms (Sako et al.; J.Con.Rel. 81 (2002) 165-172). Therein, after an initial period of incubation in the basket the dosage form to be tested is transferred into plastic tubes containing beads and left on a shaker for a suitable time of e.g. 10 minutes before it is re-transferred into the basket.

By using this method it has surprisingly been found that the burst release of indapamide following the oral administration of the composition was significantly reduced when using cellulose derivatives with a viscosity of at least 11250 cps as defined herein.

Comparative Examples employing HPMC having a viscosity of 4000 cps as used, for example, in US 5,334,392, showed a significantly higher burst effect. While HPMC having a viscosity of 4000 cps is practically contemplated in US 5,334,392, the document further mentions only indefinitely a range of viscosity from 1000 to 20000 cps. No definition of viscosity is indicated therein. According to the present invention it is possible, if desired, to exclude cellulose derivatives (and specifically HPMC) having a viscosity of 20000 cps. Instead of excluding cellulose derivatives (notably HPMC) having a viscosity of 20000 cps, in any event a combination of a cellulose derivative having a viscosity of above 11250 cps with microcrystalline cellulose can be used.

An extended release pharmaceutical composition/dosage form for oral administration comprising indapamide as active ingredient is provided. In a preferred embodiment the dosage form is a tablet. Other solid dosage forms suitable for oral administration such as pellets, granules, tablet cores or capsules are possible as well, however.

The matrix of the dosage form comprises a cellulose derivative. The cellulose derivative is preferably a cellulose ether such as methylcellulose, hydroxymethylcellulose, hydroxypropylcellulose and hydroxypropylmethylcellulose (hypromellose, HPMC) of a specified minimum viscosity. It has been found that a cellulose derivative with a viscosity equal to or above 11250 cps as determined in a 2% aqueous solution at 20 °C following the standard protocol according to the US Pharmacopeia USP29 is particularly beneficial with regard to preventing burst release.

In a preferred embodiment the cellulose derivative has a viscosity of equal to or above 11500 cps and below 140000 cps, more preferably between 12000 cps and 19500 cps. In a further preferred embodiment of the invention the nominal viscosity of the cellulose derivative is 15000 cps. Alternatively in another embodiment of the invention the nominal viscosity of the cellulose derivative is 100000 cps.

As used herein the values of viscosity are defined by determination in a 2% aqueous solution at 20 °C following the standard protocol according to the US Pharmacopeia USP29 (apparent viscosity). As typical viscosimeter suitable for viscosity measurements, the Ubbelohde type viscosimeter mentioned in USP29 can be used.

The cellulose derivatives according to the invention may comprise polymers that vary chemically and physically. Different degrees and patterns of substitution of the anhydroglucose units in the cellulose polymer are acceptable as are varying degrees of polymerization provided that a sufficiently high viscosity is achieved. One or more cellulose derivatives may be employed in pure or mixed form without affecting the effect of reducing burst release as long as a sufficiently high level of overall viscosity is maintained.

The apparent viscosity may differ from the nominal viscosity stated on the label of commercially available cellulose derivatives. In the case of HPMC, for example, apparent viscosity is supposed to be not less than 75% and not more than 140% of the nominal viscosity stated on the label (US Pharmacopoeia USP29). The apparent viscosity in the meaning of the invention shall therefore preferably be determined according to USP29 as mentioned above for each batch of cellulose derivatives to be used.

According to a preferred embodiment of the invention the cellulose derivative is HPMC, in particular HPMC of the so-called Type 2208. Commercially available mixtures having a nominal viscosity of 15000 cps, which are usually referred to as 'K15M' (e.g. Methocel K15M) have been found to prevent the burst release of indapamide in pharmaceutical compositions. Preparations of other nominal viscosities such as, for example, HPMC Type 2208 K100M having a nominal viscosity of 100000 cps may be employed as well, however, in pure form or as a mixture provided that the apparent viscosity as determined according to USP29 is equal to or above 11500 cps. The viscosity of commercially available preparations of cellulose derivatives may show some lot-to-lot variation, and therefore determination of the actial apparent viscosity as disclosed herein may be appropriate.

In a further embodiment the cellulose derivative is hydroxypropylcellulose (HPC) having a viscosity of 1% aqueous solution between 1500 and 3000 mPas using Brookfield method, which would have viscosity above 11250 cps if determined in a 2% aqueous solution at 20 °C following the standard protocol according to the US Pharmacopeia USP29. HPC is commercially available, for example, as HPC Klucel HF or Klucel HXF. HPC preparations of other nominal viscosities may be used as well, in pure form or in a mixture with cellulose derivatives of the same or another viscosity, provided that its apparent viscosity as determined according to USP29 is equal to or above 11500 cps.

In another preferred embodiment the dosage form matrix further comprises polyvinylpyrrolidone (povidone). In such a preferred embodiment, a weight ratio between HPMC and povidone is preferably between 1:0.3 and 1:0.03, preferably between 1:0.17 and 1:0.04. In a further preferred embodiment the weight ratio between HPMC and povidone is 1:0.11.

Other pharmaceutically suitable excipients known in the art such as fillers, binders, pigments, flavours and lubricants may be comprised in the dosage form. Examples of suitable fillers comprise cellulose and its derivatives, microcrystalline cellulose, lactose, sucrose, glucose, starch, pregelatinized starch, mannitol, sorbitol, calcium phosphate, calcium carbonate, dextrates, dextrins. Certain excipients may have an influence on the dissolution rate of indapamide. In a preferred embodiment one or more excipients positively interact with the cellulose derivative and have a beneficial influence on the dissolution of the pharmaceutical composition comprising indapamide or pharmaceutically acceptable salts thereof. In a particularly preferred embodiment one of the excipients is microcrystalline cellulose which can beneficially interact with HPMC of high viscosity as disclosed herein and has a beneficial influence on the dissolution of the pharmaceutical composition comprising indapamide or pharmaceutically acceptable salts thereof.

Preferred binders in the meaning of the invention comprise, for example, microcrystalline cellulose, cellulose derivatives other than those specifically disclosed herein, polyvinylpyrrolidone (povidone), carbomer such as carbopol, polymethacrylates and other polymers.

The weight ratio between HPMC and microcrystalline cellulose is preferably between is preferably between 1:2 and 1:0.07, more preferably between 1:1.2 and 1:0.1. In a specific embodiment the weight ratio between HPMC and microcrystalline cellulose is 1:0.57.

Examples of suitable lubricants according to the invention comprise magnesium stearate, calcium stearate, sodium stearyl fumarate, sodium lauryl sulphate, silica and talc.

One or more coating layers may be applied onto the pharmaceutical composition/dosage forms. Within the meaning of the present invention a coat is a layer that completely covers an object. A second active ingredient or further active ingredients may be comprised in one or more separate layers covering the dosage form comprising indapamide. A coating layer may further comprise flavours and provide for pleasant taste or taste masking. Further a coating may comprise one or more pigments providing for distinct colour of the coating layer.

An enteric coating layer may further be applied onto the dosage forms in order to protect the tablet core from gastric acid and/or direct the release to defined intestinal regions such as the duodenum or the jejunum. Enteric coatings are typically based on anionic polymers of methacrylic acid and methacrylates and are known to the person skilled in the art. Polymers suitable for enteric tablet coating are also commercially available, i.e. EUDRAGIT L 100.

The pharmaceutical composition/dosage form comprising indapamide may be used as a medicament in the non-coated as well as in the coated form. It is further possible to use the non-coated or coated pharmaceutical composition/dosage forms for combination medication, where one or more active ingredients different from indapamide are comprised in other compartments of the pharmaceutical dosage form of the present invention. Alternatively, the one or more active ingredients different from indapamide is used in a separate dosage form. Bi- or multilayer tablets may be manufactured, for example, wherein said tablet cores comprising indapamide constitute one compartment, which is associated with one or more other compartments, which may contain one or more other active ingredients. The tablet cores may, for example, be compressed into tablets or filled into capsules together with other solid dosage forms comprising other active pharmaceutical ingredients and optionally other excipients such as, for example, suitable fillers or binders. Examples of active ingredients that may be combined with the pharmaceutical compositions and tablets according to the invention comprise but are not limited to diuretics, ACE inhibitors, angiotensin II receptor antagonists, renin inhibitors, alpha blockers, beta blockers, aldosterone receptor antagonists, calcium channel blockers and vasodilators.

In a preferred embodiment the dosage form is an extended release tablet core comprising indapamide. In this preferred embodiment lactose monohydrate, povidone, HPMC with a viscosity above 11250 cps, preferably as disclosed above, as determined in a 2% aqueous solution at 20 °C following the standard protocol according to the US Pharmacopeia USP29, microcrystalline cellulose, colloidal anhydrous silica and magnesium stearate are further comprised in the tablet.

In another preferred embodiment the extended release tablet core comprising indapamide further comprises one or more other active pharmaceutical ingredients.

In one preferred embodiment the dosage form is coated for taste making. In another preferred embodiment the dosage form is a tablet core that is coated by an enteric coating layer.

In another preferred embodiment of the invention, the pharmaceutical composition/dosage form represents a multilayer tablet wherein the first layer of the multilayer tablet comprises indapamide or a pharmaceutically acceptable salt thereof and the second or further layer of the multilayer tablet represents a compartment comprising a second or further active ingredient. The release of the API from one compartment is therein independent from the release from other compartments so that either all compartments can be extended release formulations or individual compartments can be designed for immediate release of the drug while other compartments are extended release formulations.

In another preferred embodiment the pharmaceutical composition/dosage form represents a monolayer tablet wherein the monolayer represents an extended release compartment comprising indapamide or a pharmaceutically acceptable salt thereof and one or more further active ingredients.

In another preferred embodiment the dosage form is a tablet core comprising indapamide, which is compressed into a tablet together with other dosage units such as tablet cores, granules, pellets, which comprise one or more active pharmaceutical ingredients different from indapamide. The units are compressed together in presence of suitable fillers and/or binders and the tablet may optionally be coated by one or more coating layers.

In another preferred embodiment the dosage form comprising indapamide is used - alone or in combination with other drugs - as a medicament in the therapy of treatment of heart failure, hypertension, liver cirrhosis and renal diseases.

Typical daily doses suitably range from 0.1 to 5 mg, more preferably 0.5 to 3 mg that are administered orally. If pharmaceutical acceptable salts of indapamide are used, the amount of such salts to be present in the dosage form is calculated based on the amount of indapamide as defined above by considering the ratio of the molecular weight of the pharmaceutically acceptable salt of indapamide and the molecular weight of indapamide, respectively. In a preferred embodiment of the invention a tablet comprises 1.5 mg indapamide and is taken orally once daily for the treatment of heart failure and/or hypertension.

The extended-release composition of the present invention may be obtained by processes involving either direct compression of the excipients into tablets or wet or dry granulation.

In a preferred embodiment of the invention the active ingredient is blended with suitable fillers and/or binders in a granulator. The mixture is granulated with purified water or an organic solvent-water mixture and the granulate is vacuum-dried. The thus obtained granulate is then blended with the cellulose derivative of high viscosity and optionally other pharmaceutically suitable excipients such as fillers, binders and lubricants. The final blend is then compressed into tablet cores.

The term "extended release", within the meaning of the present invention, typically denotes that at least 70% of the indapamide present in the dosage form are not released before 2 hours, more preferably not before 3 hours, more preferably not before 4 hours. In accordance with beneficial and particularly preferred embodiments made possibly by the present invention, the maximum dissolution rate of indapamide following mechanical stress shall stay below 175%, preferably below 150% of the dissolution rate of indapamide measured directly before the application of mechanical stress.

Drug release profiles have been determined for a tablet comprising indapamide according to the present invention and compared to the release profiles of pharmaceutical compositions wherein HPMC of lower viscosity was used. The tests were performed using USP Apparatus 1 (basket method), where the tablets were incubated. After an initial incubation the tablets were transferred from Apparatus 1 to plastic tubes filled with medium (e.g. water) and plastic beads with a density of approximately 1 g/ml and a diameter of 8mm. In order to exert mechanical stress on the tablets said plastic tubes containing the tablets were shaken on a laboratory shaker for 10 minutes at 300 rpm. Following this treatment the tablets were again transferred to Apparatus 1 and incubation was continued. A similar method has been used by other authors (Sako et al.; J.Con.Rel. 81 (2002) 165-172).

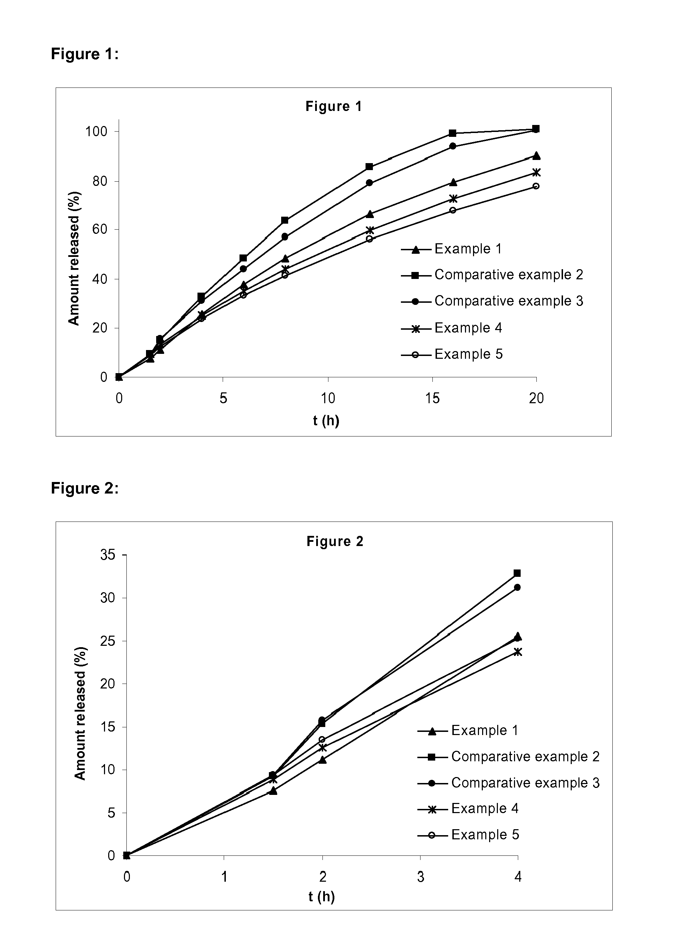

The release profiles determined by this method demonstrate that the burst release following mechanical stress (i.e. the transfer of the tablet to the plastic tubes filled with plastic beads and subsequent shaking) is significantly reduced if HPMC of high viscosity as disclosed herein is employed (see Figure 1 and 2). Similar results have been obtained using different cellulose derivative HPC but still observing viscosity characteristics of the cellulose derivative (see again Figure 1 and 2).

The dissolution profiles were used to calculate the Korsmeyer-Peppas release rate constant k and the exponent n by using the equation Q(t) = ktⁿ_{'} where Q(t) is the percentage of drug released at a given time point t. The exponent n was used as a criterion for the release mechanism. In anomalous release reactions (i.e. reactions, where release kinetics are influenced by both, dissolution of the drug and erosion of the matrix) n takes a value between 0.5 and 1, while n is 1 for zero order kinetics. Hence, with values for n approaching 1, the release profile of a drug generally becomes more linear.

The values for n were found to be 0.71 for Comparative Example 2 containing HPMC of lower viscosity and 0.79 for the composition according to the present invention (Example 1) demonstrating that the use of high viscosity HPMC as disclosed herein enables extended release under gastrointestinal mechanical stress and reduces burst release.

The impact of high viscosity HPMC as disclosed herein on burst release becomes even more apparent by inspecting the dissolution rate ΔQ/Δt after application of mechanical stress.

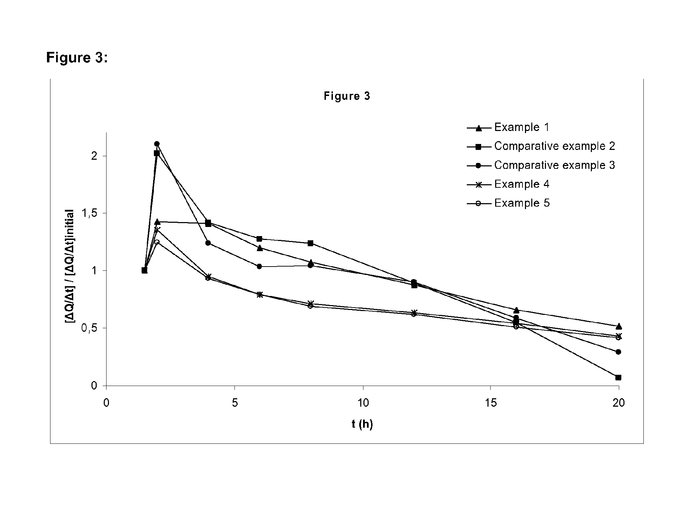

Figure 3 shows the ratio of the dissolution rates following the application of mechanical stress versus an initial dissolution rate, calculated separately for each example. The initial dissolution rate is defined as the effective dissolution rate at 1.5 hours of incubation directly before the mechanical stress treatment. In Example 1, the maximum increase in dissolution rate of 42% is significantly lower than the maximum increase of 102% in case of Comparative Example 2 containing HPMC of lower viscosity (see Table 1 ). Additionally, the graph of the dissolution rate over time is flatter in the case of Example 1 than in the case of Comparative Example 2, which confirms that the release profile of Example 1 is closer to zero order kinetics than that of Comparative Example 2. Similarly the Comparative Example 3 shows maximum increase in dissolution rate of 109%, demonstrating that a higher amount of HPMC with low viscosity does not reduces burst release. For Example 4 with HPMC having a viscosity of 100000 cps burst release is low and the maximum increase in dissolution rate is just 35%. Burst release in case of the HPC sample in Example 5 is also low. Maximum increase in dissolution rate is 25%.

**Table1:**

| | Example 1 (DQ/Dt)/(DQ/Dt)_initial | Comparative example 2 | Comparative example 3 | Example 4 | Example 5 |
|---|---|---|---|---|---|
| **t(h)** | 1 | 2 | 3 | 4 | 5 |
| 0 | / | / | / | / | / |
| **1,5** | **1** | **1** | **1** | **1** | **1** |
| **2** | **1,42** | **2,02** | **2,10** | **1,35** | **1,25** |
| 4 | 1,41 | 1,42 | 1,23 | 0,94 | 0,94 |
| 6 | 1,19 | 1,28 | 1,03 | 0,79 | 0,79 |
| 8 | 1,08 | 1,24 | 1,04 | 0,71 | 0,69 |
| 12 | 0,87 | 0,89 | 0,90 | 0,63 | 0,62 |
| 16 | 0,66 | 0,55 | 0,58 | 0,54 | 0,51 |
| 20 | 0,52 | 0,07 | 0,29 | 0,43 | 0,42 |

The present invention is described in further detail by the following examples, which are presented for illustrative purposes only and shall not be understood in any limiting manner.

### EXAMPLES

### Example 1: Preparation of indapamide ER tablets (Table 2)

| **Tablet core:** | |
|---|---|
| Indapamide | 1.500 mg |
| Lactose Monohydrate | 78.600 mg |
| Povidone | 8.000 mg |
| Purified Water^{*} | 2.467 mg |
| **Hypromellose Type 2208, viscosity 15000 cps¹** | **70.000 mg** |
| Microcrystalline Cellulose | 40.000 mg |
| Silica, Colloidal Anhydrous | 0.400 mg |
| Magnesium Stearate | 1.500 mg |
| | |
| ***Core weight*** | 200.000 mg |

| | |
|---|---|
| ^{*}Removed during manufacturing process. ¹nominal viscosity according to USP 29 Test Method | |

Amounts of indapamide and pharmaceutical excipients are listed in Table 2. Indapamide and a part of lactose monohydrate are blended and blended with the remaining quantity of lactose monohydrate and povidone in a granulator. The mixture is granulated with purified water and vacuum-dried. The granulate obtained, hypromellose, colloidal anhydrous silica and microcrystalline cellulose are sieved and blended. Sieved magnesium stearate is added to the blend obtained and blended to obtain the final mixture. The final blend is compressed into tablet cores of 200 mg.

### Comparative Example 2: Preparation of indapamide tablet using HPMC with a viscosity of 4000 cps (Table 3)

| **Tablet core:** | |
|---|---|
| Indapamide | 1.500 mg |
| Lactose Monohydrate | 78.600 mg |
| Povidone | 8.000 mg |
| Purified Water* | 2.467 mg |
| **Hypromellose Type 2208, viscosity 4000 cps¹** | **70.000 mg** |
| Microcrystalline Cellulose | 40.000 mg |
| Silica, Colloidal Anhydrous | 0.400 mg |
| Magnesium Stearate | 1.500 mg |
| | |
| ***Core weight*** | 200.000 mg |

| | |
|---|---|
| ^{*}Removed during manufacturing process. ¹nominal viscosity according to USP 29 Test Method | |

The tablets of the Comparative Example 2 are prepared as described under Example 1 with the exception of using hypromellose of lower viscosity (4000 cps, see also Table 3).

### Comparative Example 3: Preparation of indapamide tablet using high amounts of HPMC

**Table 4**

| **Tablet core:** | |
|---|---|
| Indapamide | 1.500 mg |
| Lactose Monohydrate | 78.600 mg |
| Povidone | 8.000 mg |
| Purified Water* | 2,467 mg |
| **Hypromellose Type 2208, viscosity 4000 cps¹** | **100.000 mg** |
| Microcrystalline Cellulose | 10.000 mg |
| Silica, Colloidal Anhydrous | 0.400 mg |
| Magnesium Stearate | 1.500 mg |
| | |
| ***Core weight*** | 200.000 mg |

| | |
|---|---|
| ^{*}Removed during manufacturing process. ¹nominal viscosity according to USP 29 Test Method | |

### Example 4: Preparation of indapamide tablet using HPMC (100000 cps¹) (Table 5)

| **Tablet core:** | |
|---|---|
| Indapamide | 1.500 mg |
| Lactose Monohydrate | 78.600 mg |
| Povidone | 8.000 mg |
| Purified Water* | 2,467 mg |
| **Hypromellose Type 2208, viscosity 100000 cps¹** | **70.000 mg** |
| Microcrystalline Cellulose | 40.000 mg |
| Silica, Colloidal Anhydrous | 0.400 mg |
| Magnesium Stearate | 1.500 mg |
| | |
| ***Core weight*** | 200.000 mg |

| | |
|---|---|
| ^{*}Removed during manufacturing process. ¹nominal viscosity according to USP 29 Test Method | |

### Example 5: Preparation of indapamide tablet using HPC (Table 6)

| **Tablet core:** | |
|---|---|
| Indapamide | 1.500 mg |
| Lactose Monohydrate | 78.600 mg |
| Povidone | 8.000 mg |
| Purified Water* | 2,467 mg |
| **Klucel HXF, viscosity 1500-3000 cps²** | **70.000 mg** |
| Microcrystalline Cellulose | 40.000 mg |
| Silica, Colloidal Anhydrous | 0.400 mg |
| Magnesium Stearate | 1.500 mg |
| | |
| ***Core weight*** | 200.000 mg |

| | |
|---|---|
| ^{*}Removed during manufacturing process. ²**1 % aqueous solution, Brookfield** method | |

### Example 6: Release profiles of Examples 1, 4, 5 and Comparative Examples 2 and 3

The release of indapamide from the tablets of Examples 1, 4, 5 and Comparative Examples 2 and 3, respectively, has been analysed using USP Apparatus 1 (basket method, 100 rpm). The dissolution experiments were started by incubating the tablets in the baskets of Apparatus 1 for 90 minutes. A first sample was taken for defining the initial dissolution rate before the tablets were transferred to plastic tubes containing 5 ml of media and 9 g of plastic beads with a density of approximately 1 g/ml and a diameter of 8 mm. The tablets were then shaken for 10 minutes on laboratory shaker at 300 rpm. After this manipulation, the tablets were transferred back to the baskets of Apparatus 1 and the dissolution test continued. Further samples were taken at time points at 2, 4, 6, 8, 12, 16 and 20 hours of total incubation time and the media used was water. The test was used to simulate situation in gastrointestinal tract with intense mechanical stress to show burst release effect phenomena for matrix tablets.

The graphs of the release profiles of Examples 1, 4, 5 and Comparative Examples 2 and 3 are shown in Figure 1 and Figure 2. The corresponding rates of dissolution are shown in Figure 3.

## Claims

1. An extended release pharmaceutical composition comprising indapamide as an active ingredient and a cellulose derivative, wherein the viscosity of the cellulose derivative contained in the pharmaceutical composition is equal to or above 11250 cps as determined in a 2% aqueous solution at 20 °C following the standard protocol according to the US Pharmacopeia USP29.

2. An extended release pharmaceutical composition according to claim 1 , further comprising microcrystalline cellulose.

3. An extended release pharmaceutical composition according to any of the preceding claims, wherein the cellulose derivative contained in the pharmaceutical composition has a viscosity from 11250 cps to 140000 cps, preferably from 11250 cps to 19500 cps as determined in a 2% aqueous solution at 20 °C following the standard protocol according to the US Pharmacopeia USP29.

4. The pharmaceutical composition according to any of the preceding claims, wherein the cellulose derivative is a high-viscosity grade hydroxypropylcellulose having a viscosity of 1% aqueous solution between 1500 and 3000 mPas using Brookfield method, as commercially available as Klucel Type HF or Type HXF.

5. The pharmaceutical composition according to any of the preceding claims, which is **characterized by** extended drug release under mechanical stress, wherein at least 70% of the indapamide present in the dosage form are not released before 2 hours, as determined by the basket method comprising a manipulation step for simulating mechanical stress.

6. The pharmaceutical composition according to any of the preceding claims, which is **characterized by** extended drug release following mechanical stress such that, when mechanical stress was applied for a limited period of time, the maximum dissolution rate remains less than 175%, preferably less than 150% when compared to the dissolution rate before the application of mechanical stress.

7. The pharmaceutical composition according to any of the preceding claims, wherein the relative amount of the cellulose derivative having a viscosity equal to or above 11250 cps as determined in a 2% aqueous solution at 20 °C following the standard protocol according to the US Pharmacopeia USP29, is between 20 and 70% weight/weight of the tablet core.

8. The pharmaceutical composition according to any of the preceding claims, comprising further pharmaceutical excipients selected from fillers, binders and lubricants, preferably at least one further pharmaceutical excipient is selected from the group consisting of povidone, lactose monohydrate, colloidal anhydrous silica and magnesium stearate..

9. The pharmaceutical composition according to any of the preceding claims, wherein a further excipient is povidone and the weight ratio between the cellulose derivative having a viscosity equal to or above 11250 cps as determined in a 2% aqueous solution at 20 °C following the standard protocol according to the US Pharmacopeia USP29 and povidone is between 1:0.3 and 1:0.03, preferably between 1:0.17 and 1:0.04.

10. The pharmaceutical composition according to any of the preceding claims, wherein a further excipient is microcrystalline cellulose and the weight ratio between the cellulose derivative having a viscosity equal to or above 11250 cps as determined in a 2% aqueous solution at 20 °C following the standard protocol according to the US Pharmacopeia USP29 and the microcrystalline cellulose is in a range from 1:2 to 1:0.07, preferably in a range from 1:1.2 to 1:0.1.

11. A process for manufacturing the pharmaceutical composition according to any of the preceding claims comprising mixing indapamide and a cellulose derivative having a viscosity equal to or above 11250 cps as determined in a 2% aqueous solution at 20 °C following the standard protocol according to the US Pharmacopeia USP29.

12. The process according to claim 11, wherein the cellulose derivative is blended with granules comprising indapamide as the active ingredient before compression into tablets.

13. The pharmaceutical composition according to claims 1 to 10 for use as a medicament in mammals for treatment or prophylaxis of heart failure, hypertension, liver cirrhosis and renal diseases.

14. The pharmaceutical composition according to claim 13 for use as a medicament, wherein the pharmaceutical composition is used alone or in combination with another medicament.

15. The pharmaceutical composition according to claim 14, wherein the other medicament is an antihypertensive drug.
